# EUROPÄISCHE PATENTANMELDUNG

(11) **EP 1 649 846 A1**
(43) Veröffentlichungstag der Anmeldung: **26.04.2006**
(21) Anmeldenummer: 04025080.5
(22) Anmeldetag: 21.10.2004
(51) Int. Cl.: A61K 8/18

(54) **Verwendung von Harnstoff zur Behandlung von Altersflecken**

(71) Anmelder: Neubourg Skin Care GmbH & Co. KG, 48282 Emsdetten (DE)
(72) Erfinder: Neubourg, Thomas, 59368 Werne (DE)
(74) Vertreter: Maiwald, Walter

(57) **Zusammenfassung**

Gegenstand der Erfindung ist die Verwendung von Harnstoff zur Behandlung von Altersflecken.

## Beschreibung

Gegenstand der Erfindung ist die Verwendung von Harnstoff zur Behandlung von Altersflecken.

Altersflecken sind bräunliche Flecken durch unregelmäßige Pigmentierung (Hyperpigmentierung) der Haut, die auf den Handrücken, an Unterarmen oder im Gesicht und Dekollete auftreten. Sie entwickeln sich durch eine über Jahre erfolgte Sonnenbestrahlung der Haut, unter umständen auch oder verstärkt durch Umwelteinflusse wie das Rauchen. Es handelt sich um irreversible Veränderungen. Altersflecken müssen nicht medizinisch behandelt werden; sie sind keine Krankheit. Oft beeinträchtigen Sie jedoch das Erscheinungsbild erheblich und können so zu einem seelischen Problem werden.

Die kosmetische Entfernung der Altersflecken erfolgt üblicherweise beim Hautarzt. Da es sich um ein kosmetisches und nicht medizinisches Problem handelt, müssen die Behandlungskosten selbst getragen werden.

Ein übliches Behandlungsverfahren ist die Lasertherapie, die aufwendig und teuer ist.

Ergänzend und alternativ werden hydrochinonhaltige Cremes verabreicht.

Eine weitere Therapie verwendet Tretinoin und alpha-Hydroxysäuren. Die Verwendung eines Komplexes aus α-Hydroxysäuren in Verbindung mit Harnstoff zur Behandlung verschiedener Hautkrankheiten wird in der WO 03/086291 offenbart.

Die WO 03/026680 offenbart die Behandlung von altersbedingten Hautveränderungen mit basischen Formulierungen mit einem pH-Wert von 8 bis 13.

Der Erfindung liegt die Aufgabe zugrunde, eine Therapiemöglichkeit zur Verfügung zu stellen, die in hohem Masse wirksam ist, auf einfache und kostengünstige Weise vom Patienten selbst appliziert werden kann, und vom Patienten in hohem Masse und auch zur Behandlung über längere Zeiträume akzeptiert wird.

Das der Erfindung zugrunde liegende Problem wird überraschenderweise gelöst durch die Verwendung von Harnstoff zur Behandlung von Altersflecken nach einem der Ansprüche 1 bis 8. Der Harnstoff wird in einer geeigneten Zubereitung bereitgestellt und verwendet.

Vorzugsweise weist die Zubereitung einen pH-Wert kleiner 8,0 auf. Der pH-Wert sollte hautverträglich sein. In bevorzugten Ausführungsformen der Erfindung ist der pH-Wert kleiner als 7,9 oder 7,8 oder 7,5. In bevorzugten Ausführungsformen ist der pH-Wert größer als 5, größer als 6 oder größer als 6,5.

Die erfindungsgemäß zu verwendende Zubereitung enthält vorzugsweise mehr als 3 Gew. %, insbesondere 5 bis 50 Gew. % Harnstoff. In weiteren Ausführungsformen der Erfindung enthält sie 5 bis 40 oder 10 bis 30 Gew. % Harnstoff.

In einer bevorzugten Ausführungsform enthält die Zubereitung nicht eine Substanz, die eine Hydroxygruppe, insbesondere eine α-Hydroxysäure und eine Carboxygruppe enthält.

Die erfindungsgemäße Verwendung von Harnstoff wird durch den bekannten Stand der Technik nicht offenbart oder nahegelegt. In der WO 03/026680 werden Formulierungen zur Behandlung von altersbedingten Veränderungen der Haut offenbart, die einen pH-Wert von 8 bis 13 aufweisen müssen. Zur Einstellung des pH-Werts wird eine Vielzahl von organischen und anorganischen Basen benannt (z. B. Seiten 10-12), unter denen auch Harnstoff aufgelistet ist. In den zahlreichen Ausführungsbeispielen wird Harnstoff nicht verwendet. Soweit die Beispiele die Behandlung von Altersflecken betreffen, wird eine aktive Substanz wie Hydrochinon zugesetzt. Daher offenbart die WO 03/026680 allenfalls die Verwendung von Harnstoff zur Einstellung des pH-Werts, nicht jedoch eine Indikation gemäß der vorliegenden Erfindung.

Der Gegenstand der Erfindung ist auch neu und überraschend angesichts der WO 03/086291. Dort wird die Verwendung eines Komplexes von Harnstoff mit einer Hydroxycarbonsäure zur Behandlung einer Vielzahl von Hautkrankheiten und -veränderungen offenbart. Die Hydroxycarbonsäure wird dabei als die "funktionelle" Substanz bezeichnet (siehe z. B. Absatz 16). Es wird daher nicht offenbart, dass Harnstoff alleine eine besondere Wirksamkeit gegen Altersflecken aufweisen könnte, und wegen der Fokussierung auf den Komplex mit der funktionalen α-Hydroxysäure ist es auch überraschend, dass Harnstoff alleine eine hohe Wirksamkeit aufweist. Die Verwendungen gemäß der Erfindung der WO 03/086291 sind nicht Gegenstand der vorliegenden Erfindung.

Zum Stand der Technik gehören weitere Publikationen, die die Verwendung von bestimmten funktionellen Substanzen offenbaren. In diesen Publikation wird häufig Harnstoff als üblicher Zusatzstoff aufgelistet, ohne dass eine Wirksamkeit von Harnstoff beschrieben wird, so dass der Gegenstand der vorliegenden Erfindung nicht nahegelegt wird.

In einer bevorzugten Ausführungsform der Erfindung erfolgt daher keine gleichzeitige Verwendung weiterer funktionaler Substanzen mit Wirksamkeit gegen Altersflecken, wie z. B. von α-Hydroxycarbonsäuren oder Hydrochinonen.

Erfindungsgemäß kann der Harnstoff in Form einer Lösung, einer Creme, einer Salbe, einer Schaumcreme oder auch einer Lotion bereitgestellt werden.

In bevorzugten Ausführungsformen wird der Harnstoff in einer Zubereitung bereitgestellt, die zusätzlich Emulgatoren, Fettsäuren, Coemulgatoren, Moisturizer, Hautpflegeadditive, Salze, Wasser, rückfettende Stoffe und/oder Konservierungsmittel enthält.

In einer Ausführungsform der Erfindung ist die Zubereitung eine Schaumcreme (gleichbedeutend mit Cremeschaum, Schaumhautcreme). Schaumcremes sind stabile Aerosole, wie sie zum Beispiel in der WO 99/08649 beschrieben werden.

Im Grundprinzip handelt es sich um emulsoide Zweiphasensysteme mit hydrophilen und hydrophoben bzw. lipophilen Komponenten, die auf der Haut ein zweidimensionales Netzwerk bilden. Dieses Netzwerk ist in der Lage, die Haut vor äußeren Einflüssen zu schützen, zugleich aber auch die Schweißabdunstung der Haut weiter zu gestatten. In der WO 99/08649 werden auch harnstoffhaltige Schaumcremes offenbart. Auf das dort beschriebene Herstellungsverfahren wird hier ausdrücklich verwiesen.

Eine erfindungsgemäß verwendbare Schaumhautcreme enthält beispielsweise 4 bis 15 Gew. % Öl in Wasser Emulgator, 1 bis 10 Gew. % Fettsäure, 0.4 bis 2.3 Gew. % Coemulgator, 1 bis 10 Gew. % Moisturizer, 0.05 bis 1 Gew. % Hautpflegemittel, 3 bis 50 % Gew. Harnstoff sowie Wasser bis zum Ausgleich auf 100 Gew. %.

### Ausführungsbeispiel :

Untersucht wurden insgesamt 16 Patienten. Es handelt sich um 12 Frauen im Alters zwischen 53 und 65 Jahren. Davon waren 8 mit atopischer Dermatitis in Behandlung und 4 mit irritativer Dermatitis bei *Xerosis cutis.* Ferner waren 4 Männer im Alter zwischen 55 und 63 Jahren mit einbezogen; davon hatten 2 atopische Dermatitis und 2 eine irritative Dermatitls bei *Xerosis cutis.*

Der Untersuchungszeltraum umfasste insgesamt 10 Wochen mit 14tägigen Kontrollen. Dabei wurden die Hände und Unterarme auf der einen Seite mit harnstoffihaltigem Cremeschaum A, auf der anderen Seite mit Vergleichscremeschaum B ohne Harnstoff 3x täglich behandelt. Zusätzliche Lokaltherapie erfolgte an diesen Stellen nicht. Besonders dunkle und subjektiv störende Altersflecken an den Unterarmen wurde alle 2 Wochen melaninometrisch untersucht (Mexameter, Courage+ Khazaka, Köln) und deren Melanin-Wert notiert. Ferner nahmen die Patienten eine Selbstbeurteilung der Flecken vor anhand einer Intensitätsskala von 10 ("sehr dunkel") bis 0 ("vollständig abgeblasst").

Bei den verwendeten Cremeschäumen A und B handelt es sich um die im Handel erhältlichen Produkte Allpresan® -3-Cremeschaum und Allpresan® -1-Cremeschaum der Allpresan GmbH und Co. KG, 48282, Emsdetten, DE.

Cremeschaum A (Allpresan-3-Cremeschaum) enthält Wasser, 10 % Harnstoff, Decyloleat, Butan, Stearinsäure, Cetearylalkohol, Propan, Propylenglykol, Glyerin, Glycerylstearat, Allantoin, Natriumlauroylsarcosinat, Dimethicon, Triceteareth-4-phosphat, Paraffinum Liquidum und Aminomethylpropanol.

Cremeschaum B (Allpresan-1-Cremeschaum) enthält Wasser, Butan, Stearinsäure, Cetearylalkohol, Propan, Propylenglykol, Glycerin, Glycerylstearat, Allantoin, Decyloleat, Octyldodecanol, Natriumlauroylsarcosinat, Dimethicon, Tricetheareth-4-Phosphat, Paraffinum Liquidum und Aminomethylpropanol.

Die Cremeschäume sind nach bekannten Verfahren herstellbar, wie sie z. B. offenbart werden in der WO 99/08649.

### Ergebnisse:

| Melaninometrie (Melanin-Wert ohne Dimension): | | |
|---|---|---|
| Allpresan-1 -Cremeschaum: | initial: | 510 ± 9 |
| | nach 10 Wochen: | 506 ±7 |
| Allpresan-3-Cremeschaum: | initial: | 512 ± 11 |
| | nach 10 Wochen: | 461 ±10 |

| Patientenselbstbeurteilung (Punktzahl): | | |
|---|---|---|
| Allpresan-1-Cremeschaum: | initial: | 9 ± 1 |
| | nach 10 Wochen: | 8 ± 1 |
| Allpresan-3-Cremeschaum: | initial: | 9 ± 1 |
| | nach 10 Wochen: | 2 ± 1 |

### Beurteilung:

Objektiv lässt sich melaninometrisch ein signifikanter Abfall der Werte unter Cremeschaum A feststellen, während sich unter Cremeschaum B keine wesentliche Veränderung erkennen lässt. In der Selbstbeurteilung der Patienten lässt sich ein gleiches Bild feststellen. Unter Cremeschaum B sind keine nennenswerten Veränderungen in der Farbintensität der Altersflecken erkennbar. Unter Verwendung von harnstoffhaltigem Cremeschaum A kommt es dagegen zu einer deutlichen Aufhellung, bei einigen Patienten sogar zum völligen Verschwinden der Farbverschiebungen. Auffällig war der hohe Zufriedenheitsgrad und die Neigung, andere Körperregionen mit Altersflecken weiter mit Cremeschaum A zu behandeln. Angesichts der deutlichen Effekte ist davon auszugehen, dass der Harnstoff zu einem Abtragen der Pigmentverschiebungen in der Hornschicht geführt hat.

## Patentansprüche

1. Verwendung von Harnstoff zur Behandlung von Altersflecken.

2. Verwendung nach Anspruch 1, **dadurch gekennzeichnet, dass** der Harnstoff in einer Zubereitung bereitgestellt wird, die mehr als 3%, insbesondere 5 bis 50 % Harnstoff enthält.

3. Verwendung nach Anspruch 1 oder 2, **dadurch gekennzeichnet, dass** der Harnstoff in Form einer Lösung, einer Creme, einer Schaumcreme, einer Salbe oder einer Lotion bereitgestellt wird.

4. Verwendung nach einem der Ansprüche 1 bis 3, **dadurch gekennzeichnet, dass** der Harnstoff in einer Zubereitung bereitgestellt wird, die zusätzlich Emulgatoren, Fettsäuren, Coemulgatoren, Moisturizer, Hautpflege-Additive, Salze, Wasser, rückfettende Stoffe, Konservierungsmittel enthält.

5. Verwendung nach Anspruch einem der Ansprüche 3 oder 4, **dadurch gekennzeichnet, dass** die Schaumcreme 4 bis 15 Gew. % Öl in Wasser Emulgator, 1 bis 10 Gew. % Fettsäure, 0.4 bis 2.3 Gew. % Coemulgator, 1 bis 10 Gew. % Moisturizer, 0.05 bis 1 Gew. % Hautpflegemittel, 3 bis 50 % Harnstoff sowie Wasser bis zum Ausgleich auf 100 Gew. % enthält.

6. Verwendung nach einem der Ansprüche 1 bis 5, **dadurch gekennzeichnet, dass** der Harnstoff in einer Zubereitung bereitgestellt wird, die einen pH-Wert kleiner 8,0 aufweist.

7. Verwendung nach einem der Ansprüche 1 bis 6, **dadurch gekennzeichnet, dass** der Harnstoff in einer Zubereitung bereitgestellt wird, die nicht zusätzlich eine Substanz enthält, die eine Hydroxygruppe und eine Carboxygruppe aufweist.

8. Verwendung nach einem der Ansprüche 1 bis 7, **dadurch gekennzeichnet, dass** die Zubereitung keine weitere Substanz mit Wirksamkeit gegen Altersflecken enthält.
